(19) Europäisches Patentamt
European Patent Office
Office européen des brevets

(11) **EP 3 894 158 B1**

(12) **FASCICULE DE BREVET EUROPEEN**

(45) Date de publication et mention de la délivrance du brevet:
**04.10.2023 Bulletin 2023/40**

(21) Numéro de dépôt: **19817350.2**

(22) Date de dépôt: **13.12.2019**

(51) Classification Internationale des Brevets (IPC):
**B28D 5/00** *(2006.01)*  **B28D 5/04** *(2006.01)*
**A61B 5/00** *(2006.01)*  **G05B 19/4065** *(2006.01)*

(52) Classification Coopérative des Brevets (CPC):
**B28D 5/045; A61B 5/7239; B28D 5/0064;**
**G05B 19/404;** G05B 2219/45042

(86) Numéro de dépôt international:
**PCT/EP2019/085187**

(87) Numéro de publication internationale:
**WO 2020/120777 (18.06.2020 Gazette 2020/25)**

(54) **PROCEDÉS DE SUIVI DE LA COUPE EN FONCTION DE LA FLÈCHE D'AU MOINS UN FIL DE COUPE ET DISPOSITIF ASSOCIÉ**

VERFAHREN ZUR SCHNEIDVERFOLGUNG JE NACH DEM BOGEN VON MINDESTENS EINER SCHNITTLINIE UND ZUGEHÖRIGE VORRICHTUNG

MONITORING PROCESSES OF CUTTING AS A FUNCTION OF A WIRE BOW OF AT LEAST ONE CUTTING WIRE AND ASSOCIATED DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.12.2018 FR 1872946**

(43) Date de publication de la demande:
**20.10.2021 Bulletin 2021/42**

(73) Titulaire: **Commissariat à l'Energie Atomique et aux Energies Alternatives**
**75015 Paris (FR)**

(72) Inventeurs:
• **RIVA, Roland**
**38054 GRENOBLE CEDEX 09 (FR)**
• **COUSTIER, Fabrice**
**38054 GRENOBLE CEDEX 09 (FR)**
• **VELET, Nicolas**
**38054 GRENOBLE CEDEX 09 (FR)**

(74) Mandataire: **Cabinet Camus Lebkiri**
**25 rue de Maubeuge**
**75009 Paris (FR)**

(56) Documents cités:
**EP-A1- 2 708 342    EP-A2- 0 079 801**
**WO-A1-2014/167392    WO-A2-03/054503**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

## DOMAINE TECHNIQUE DE L'INVENTION

Le domaine technique de l'invention est celui de la coupe à l'aide de fils. La présente invention concerne un procédé de suivi de la coupe en fonction de la flèche d'au moins un fil de coupe ainsi que le dispositif associé.

## ARRIERE-PLAN TECHNOLOGIQUE DE L'INVENTION

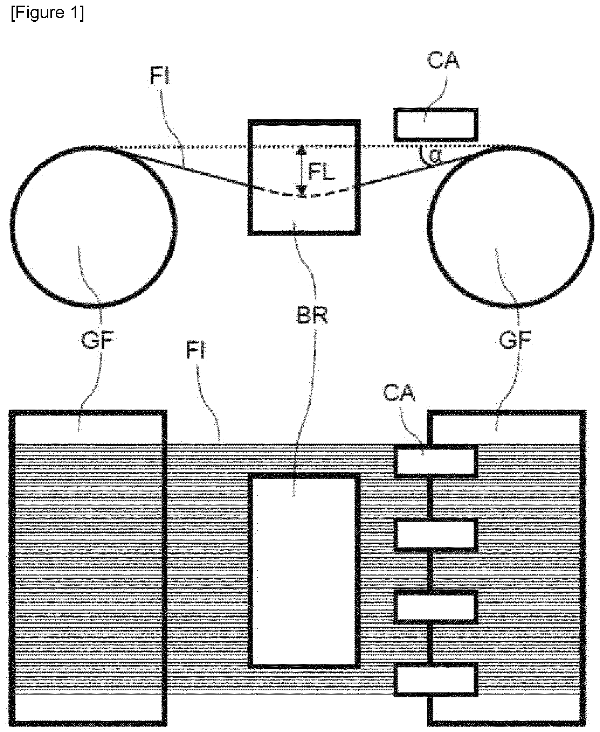

Le sciage en tranches fines du silicium ou d'autres substances dures telles que le saphir, pour obtenir des wafers, peut être réalisé à l'aide de fils lisses ou structurés entraînant des grains de SiC en suspension dans un liquide (en anglais slurry) ou de fils à abrasifs liés (diamants, le plus souvent) de quelques dizaines de $\mu$m de diamètre. La découpe résulte alors d'un mouvement longitudinal du fil, éventuellement alternatif (inversion périodique de sens), sur lequel l'objet à scier exerce une force d'appui, ou inversement. Le fil, qu'il soit unique ou multiple, prend alors une « flèche » (figure 1), qui dépend des conditions (tension, vitesse et taux de renouvellement du fil, vitesse de pénétration dans l'objet découpé, lubrification) et de la difficulté de sciage (nature et qualité du matériau). Cette flèche (*bow* en anglais) correspond au déplacement maximum du fil sous l'effet de la charge ou, de manière équivalente, à son angle par rapport à sa position au repos ($\alpha$ sur la figure 1). Dans la suite, la notion de flèche fera référence à l'angle $\alpha$ et sera parfois appelée « flèche angulaire ». En effet, la « flèche angulaire » apparaît plus générique, car elle ne dépend pas de la configuration géométrique (entraxe et diamètre des guide-fils). De manière générale, la relation entre « flèche » et « flèche angulaire » est donnée par la formule suivante :

$$\alpha = \tan^{-1}\left(\frac{2 \times \text{FL}}{\text{ENTR}}\right)$$

où FL est la valeur de la flèche et ENTR est la valeur de l'entraxe entre les guide fils (les deux valeurs étant exprimées dans la même unité de longueur).

L'opération de sciage est classiquement réalisée dans des scies où le fil abrasif est enroulé plusieurs centaines de fois autour de guides cylindriques rotatifs rainurés qui déterminent l'écartement entre les passes, pour former une nappe (voir partie inférieure de la figure 1). Dans le cas des wafers de silicium pour applications photovoltaïques, pour lesquels le procédé à fil diamanté (abrasif lié) est désormais majoritairement utilisé, l'écartement entre passes est de 150 à 300 $\mu$m, afin d'obtenir des plaquettes d'environ 180 $\mu$m d'épaisseur.

Chaque coupe met en oeuvre plusieurs centaines de mètres de fil, voire plusieurs kilomètres. Ce dernier effectue des allers-retours dans la brique avec un faible renouvellement à chaque cycle (de l'ordre de 1% au maximum). Actuellement, le sciage d'un bloc de 156 mm de côté s'effectue en moins de 3 heures par cette technique (contre 8 heures au slurry), avec une consommation équivalente de 1 à 3 mètres par wafer, suivant la qualité du matériau. La casse du fil durant cette opération affecte fortement la productivité (perte de temps et de matière) et il est donc préférable de maintenir son occurrence en dessous de 2%.

La flèche prise par le fil durant la découpe témoigne de sa « souffrance » et doit rester dans certaines limites. La surveillance de ce paramètre est donc importante pour mener la coupe à terme dans des conditions satisfaisantes. De nombreuses solutions pour mesurer la flèche du fil dans une scie en fonctionnement ont été proposées, généralement en plusieurs points de la nappe. De telles solutions utilisent des capteurs ultrasonores, inductifs, capacitifs, LASER ou de contact, fixes ou déplaçables, éventuellement protégés de l'environnement de la chambre de coupe par des enveloppes. Par exemple, dans la solution décrite dans le document EP 2583778 A1 « *Method and apparatus for measuring wire-web bow in a wire saw* » et US 6178961 B1 « *Wire saw control method and wire saw* », la valeur de la charge (c'est-à-dire la force dans la direction du déplacement de la brique) est déduite de la mesure de flèche, connaissant la tension du fil et la géométrie du dispositif. Certaines solutions intègrent également une contreréaction sur les paramètres de coupe lorsque la valeur de la flèche dépasse un seuil qui varie généralement entre 5,5 mm et 10 mm, suivant les méthodes. La contreréaction peut par exemple, en premier lieu, consister en une réduction de la vitesse de table de 10 à 20%, éventuellement accompagnée d'une augmentation de la vitesse du fil, voire du débit de lubrification dans le cas d'une découpe au slurry. La contreréaction peut aller jusqu'à l'arrêt de la coupe si les conditions le nécessitent.

Le document EP 2708342 A1 « *Wire bow monitoring system for a wire saw* » divulgue un procédé de suivi de la coupe, la coupe étant effectuée avec un mouvement aller-retour du fil de coupe, le mouvement aller-retour du fil étant effectué à une fréquence, le suivi étant effectué en fonction de la flèche d'au moins un fil de coupe à l'aide d'au moins un capteur, le fil présentant une flèche lors de la coupe, ladite flèche étant caractérisée par un angle, le procédé comprenant, pour chaque capteur une étape de mesure de la flèche a du fil.

Ce document décrit aussi un procédé de suivi de la coupe en fonction de la flèche d'au moins deux fils de coupe à l'aide d'au moins deux capteurs, le fil présentant une flèche lors de la coupe, ladite flèche pouvant être caractérisée par un angle, le procédé de suivi comprenant, pour chaque capteur une étape de mesure de la flèche a du fil.

Ce document décrit en particulier une mesure de flèche d'un ou plusieurs fils (nappe) dans une scie de découpe de matériaux semi-conducteurs (procédé au slurry ou à fil diamanté) au moyen d'un arrangement de capteurs inductifs, capacitifs ou à contact. Les capteurs peuvent être analogiques, avec une sortie distance - tension continue et linéaire, ou numériques, délivrant une valeur « 0 » si la flèche est inférieure à un seuil compris entre 0,1 et 1,0 mm, voire 0,2 et 0,6 mm, et « 1 » au-dessus. Afin de maintenir la précision de détection, au moins un capteur peut être déplaçable au moyen d'un actuateur, voire pivotable. Ce document propose des critères de surveillance de la flèche en prenant l'exemple d'un dispositif à 4 capteurs disposés le long d'un fil : le passage de l'état « 0 » à l'état « 1 » d'un ou plusieurs capteurs est interprété en termes de déviation angulaire (inférieure à 2° si tous les capteurs restent à « 0 », supérieure à 8° si 4 capteurs sur 4 sont à « 1 ») ou de déflection (déplacement par rapport à la position au repos ; < 3 mm si tous les capteurs restent à « 0 », > 12 mm si 4 capteurs sur 4 sont à « 1 ») . Sur cette base, une méthode de suivi est établie. Aucune action n'est requise sur les paramètres de coupe si l'angle est inférieur à 6° ou, de manière équivalente, pour un déplacement du fil inférieur à 9 mm. Au-dessus de ces valeurs, la vitesse du fil et/ou la vitesse de coupe (table) doivent être réduites de 10%. Si elles dépassent 8° (ou 12 mm), la vitesse du fil doit être encore augmentée de 20% et celle de coupe diminuée ou, de manière alternative, la coupe doit être interrompue et/ou un opérateur alerté.

Le document JP 2003 127058 A « *Wire saw* » s'appuie quant à lui sur une mesure de flèche par sonde capacitive déplaçable dans la direction perpendiculaire à la nappe. Il introduit une action sur la vitesse de table, la vitesse du fil ou le débit de slurry pour que la flèche reste inférieure ou égale à 10 mm. D'après les auteurs, le maintien de la flèche au voisinage de cette valeur permet d'obtenir des wafers de bonne qualité en termes d'épaisseur, de variation d'épaisseur (TTV - Total Thickness Variation) et de voilage (warp).

De même, le document CN 202428570 U « *Multi-wire cutting machine with wire arc detectors* » met en oeuvre des capteurs de proximité capacitifs (6-10 par ligne) intégrés dans des baffles situés sous la nappe, au voisinage des guide-fils. Un avertissement est émis lorsque la flèche atteint 7 mm et la table est arrêtée à 12 mm.

Enfin, le document CN 202507408 U « *Intelligent device for automatically adjusting speed of cutting silicon pièces with diamond wire* » présente un asservissement réduisant la vitesse de table lorsque la flèche atteint 5,5 mm sur une nappe de fils diamantés.

Comme cela vient d'être exposé, le suivi de la coupe dans les procédés de l'art antérieur s'appuie seulement sur la surveillance d'une ou de plusieurs valeurs instantanées de la flèche du fil (ou, de manière équivalente, de la charge qui lui est appliquée), ce qui limite la capacité d'anticipation.

Il existe donc un besoin d'un procédé de suivi de la coupe permettant d'anticiper les incidents de coupe afin de corriger les paramètres de sciage.

## RESUME DE L'INVENTION

L'invention offre une solution aux problèmes évoqués précédemment, en effectuant un suivi de la coupe non pas seulement en fonction de la flèche, mais également en fonction de la dérivée temporelle de celle-ci.

Un premier aspect de l'invention concerne un procédé de suivi de la coupe en fonction de la flèche d'au moins un fil de coupe, de préférence une pluralité de fils, à l'aide d'au moins un capteur, de préférence une pluralité de capteurs, le fil présentant une flèche lors de la coupe, ladite flèche étant caractérisée par un angle $\alpha$, le procédé comprenant, pour chaque capteur :

- une étape de mesure de la flèche $\alpha$ du fil ;
- une étape de détermination de la dérivée temporelle première $\dot{\alpha}$ de ladite flèche $\alpha$ à partir de la mesure lissée.

De plus, ces étapes sont réitérées à intervalles réguliers de sorte à assurer un suivi de la coupe.

Grâce à l'invention, il est possible d'anticiper les évolutions des conditions de coupe, le suivi ne se faisant plus seulement sur la mesure de la flèche, mais également à partir de la dérivée temporelle première $\dot{\alpha}$ de ladite flèche $\alpha$.

Outre les caractéristiques qui viennent d'être évoquées dans les trois paragraphes précédents, le procédé selon un premier aspect de l'invention peut présenter une ou plusieurs caractéristiques complémentaires parmi les suivantes, considérées individuellement ou selon toutes les combinaisons techniquement possibles.

Dans un mode de réalisation, le procédé comprend une étape de traitement de la mesure de la flèche, la détermination de la dérivée temporelle première $\dot{\alpha}$ de la flèche $\alpha$ se faisant à partir de la mesure traitée.

Ainsi, les données de mesures peuvent être traitées de sorte à rendre les calculs de dérivée temporelle plus précis, par exemple en lissant le signal mesuré par le ou les capteurs.

Dans un mode de réalisation, la coupe est effectuée avec un mouvement aller-retour du fil de coupe, le mouvement aller-retour du fil étant effectué à une fréquence déterminée et l'étape de traitement de la mesure de la flèche comprend le lissage de la mesure issue du capteur, ledit lissage étant effectué en fonction de la fréquence du mouvement aller-retour du fil, la détermination de la dérivée temporelle première $\dot{\alpha}$ de la flèche $\alpha$ se faisant à partir de la mesure lissée.

Ainsi, le traitement du signal prend en compte le mouvement d'aller-retour du fil ce qui permet de réduire le bruit de mesure et donc la précision du suivi.

Dans un mode de réalisation, le procédé selon un premier aspect de l'invention comprend en outre une étape d'établissement d'un statut de la coupe en fonction de la flèche $\alpha$ du fil et de la dérivée temporelle première $\dot{\alpha}$ de ladite flèche $\alpha$ mesurée par chaque capteur.

Ainsi, le procédé selon l'invention propose une vision synthétique de l'état de la coupe à travers l'utilisation de statuts.

Dans un mode de réalisation, le statut de la coupe est considéré comme « nominal » si, pour chaque capteur CA, $\alpha$ est inférieur à un premier seuil $S_{1*}^{\alpha}$ et si, pour chaque capteur CA, $\dot{\alpha}$ est inférieur à un deuxième seuil $S_{2*}^{\dot{\alpha}}$ lorsque la coupe est avancée à plus de 15%.

Dans un mode de réalisation, le statut de la coupe est considéré comme « à risque » si, pour un et un seul capteur, $\alpha$ est supérieur à un premier seuil $S_{1*}^{\alpha}$, et si, pour un nombre de capteurs prédéterminé, $\dot{\alpha}$ est supérieur à un deuxième seuil $S_{2*}^{\dot{\alpha}}$ et inférieur à un troisième seuil $S_{3*}^{\dot{\alpha}}$ lorsque la coupe est avancée à plus de 15%.

Dans un mode de réalisation, le statut de la coupe est considéré comme « dangereux » lorsque, pour au moins deux capteurs, $\alpha$ est supérieur à un premier seuil $S_{1*}^{\alpha}$ ou lorsque, pour au moins deux capteurs, $\dot{\alpha}$ est supérieur à un troisième seuil $S_{3*}^{\dot{\alpha}}$ lorsque la coupe est avancée à plus de 10%.

Ainsi, il est possible de déterminer rapidement si une coupe présente ou non un risque d'incident et de modifier, le cas échéant, les paramètres de coupe. De préférence on entend par avancement de la coupe le rapport du temps écoulé sur le temps total théorique de coupe, ce dernier étant toujours connu à l'avance et défini dans la recette de coupe. Cependant, l'avancement de la coupe pourra également être défini en fonction de position de la table à l'instant considéré, de la position de la table au début de la coupe et de la position supposée de la table en fin de coupe.

Dans un mode de réalisation, le procédé selon un premier aspect de l'invention comprend une étape de détermination de la dérivée temporelle seconde $\ddot{\alpha}$ de la flèche. De plus, lorsque la mesure est traitée, la dérivée temporelle seconde $\ddot{\alpha}$ de la flèche est calculé à partir de la mesure traitée (par exemple lissée).

Dans un mode de réalisation, l'étape d'établissement d'un statut de la coupe prend également en compte la dérivée temporelle seconde $\ddot{\alpha}$ de la flèche $\alpha$ mesurée par chaque capteur.

Dans un mode de réalisation, le statut de la coupe est « nominal » si, pour chaque capteur, $\alpha$ est inférieur à un premier seuil $S_1^{\alpha}$ et si, pour chaque capteur, $\ddot{\alpha}$ est inférieur à un deuxième seuil $S_2^{\ddot{\alpha}}$.

Dans un mode de réalisation, le statut de la coupe est « à risque » si, pour un et un seul capteur, $\alpha$ est supérieur à un premier seuil $S_1^{\alpha}$, et si, pour un nombre de capteurs prédéterminé, $\ddot{\alpha}$ est inférieur à un troisième seuil $S_3^{\ddot{\alpha}}$, et, lorsque la coupe est avancée au-delà d'un seuil d'avancement prédéterminée, par exemple avancée à plus de 15%, si, pour au moins un capteur, $\dot{\alpha}$ est supérieur à un quatrième seuil $S_4^{\dot{\alpha}}$.

Dans un mode de réalisation, le statut de la coupe est « dangereux » lorsque, pour au moins deux capteurs, $\alpha$ est supérieur à un premier seuil $S_1^{\alpha}$ ou lorsque, pour au moins deux capteurs, $\ddot{\alpha}$ est supérieur à un deuxième seuil $S_2^{\ddot{\alpha}}$ et si, pour ces deux capteurs, $\dot{\alpha}$ est supérieur à un cinquième seuil $S_5^{\dot{\alpha}}$.

Ainsi, il est possible de déterminer rapidement si une coupe présente ou non un risque d'incident et de modifier, le cas échéant, les paramètres de coupe.

Dans un mode de réalisation, le procédé selon un premier aspect de l'invention comprend une étape de correction des paramètres de la coupe et/ou une étape d'émission d'une alerte en fonction du statut de la coupe.

Ainsi, les paramètres de la coupe peuvent être modifiés durant le suivi afin de prévenir tout incident de coupe.

Dans un mode de réalisation, le procédé selon un premier aspect de l'invention comprend une étape de détermination du rapport entre la flèche et l'avancement de la coupe ou le temps de coupe, le suivi de la coupe étant également fonction de la valeur dudit rapport.

Ainsi, en plus de la dérivée temporelle de la flèche, le suivi de la coupe peut également prendre en compte le rapport entre la flèche et l'avancement de la coupe ou du temps de coupe, permettant la détection d'éventuelles anomalies qui n'apparaitraient pas dans la dérivée temporelle.

Un deuxième aspect de l'invention concerne un dispositif de suivi de la coupe comprenant des moyens pour mettre en oeuvre un procédé selon un premier aspect de l'invention.

Un troisième aspect de l'invention concerne un programme d'ordinateur comprenant des instructions qui conduisent le dispositif selon un deuxième aspect de l'invention à exécuter les étapes du procédé selon un premier aspect de l'invention.

Un quatrième aspect de l'invention concerne un support lisible par ordinateur, sur lequel est enregistré le programme d'ordinateur selon un troisième aspect de l'invention.

L'invention et ses différentes applications seront mieux comprises à la lecture de la description qui suit et à l'examen des figures qui l'accompagnent.

## BREVE DESCRIPTION DES FIGURES

Les figures sont présentées à titre indicatif et nullement limitatif de l'invention.

La figure 1 montre une représentation schématique d'un procédé de découpe dont le suivi peut être assuré avec un procédé selon un premier aspect de l'invention.

La figure 2 montre un ordinogramme d'un mode de réalisation d'un procédé selon un premier aspect de l'invention.

La figure 3 montre l'évolution de la flèche au cours de la coupe mesurée par quatre capteurs différents.

La figure 4 montre le principe de lissage de la flèche et de sa dérivée.

La figure 5A montre l'évolution de la flèche au cours de la coupe mesurée par les capteurs puis lissée au moyen d'une méthode de traitement du signal.

La figure 5B montre l'influence du nombre de période prise en compte pour le lissage sur la mesure de la flèche lissée.

La figure 6A montre l'évolution de la dérivée temporelle première de la flèche au cours de la coupe calculée à partir des mesures lissées.

La figure 6B montre l'influence du nombre de périodes prises en compte pour le lissage sur la détermination de la dérivée temporelle première de la flèche.

La figure 7 montre l'évolution de la flèche au cours de la coupe mesurée par différents capteurs lors de quatre coupes différentes.

La figure 8 montre l'évolution de la dérivée temporelle première de la flèche au cours de la coupe calculée à partir de la flèche lissée, pour différents capteurs lors de quatre coupes différentes.

La figure 9 montre l'évolution lors de la coupe du rapport entre la valeur de la flèche et l'avancement de la coupe pour différents capteurs lors de quatre coupes différentes.

## DESCRIPTION DETAILLEE D'AU MOINS UN MODE DE REALISATION DE L'INVENTION

Sauf précision contraire, un même élément apparaissant sur des figures différentes présente une référence unique. Le procédé selon un premier ou un deuxième aspect de l'invention ne fait aucune hypothèse sur la nature du fil utilisé ou bien encore sur le type d'objets découpés. Aussi, bien que les exemples donnés dans la suite soient relatifs à des fils de coupe à abrasifs fixes, les enseignements de la présente invention sont applicables à tout procédé de coupe utilisant un fil ou des fils dont le déroulé peut être suivi par l'intermédiaire de la flèche dudit fil ou desdits fils.

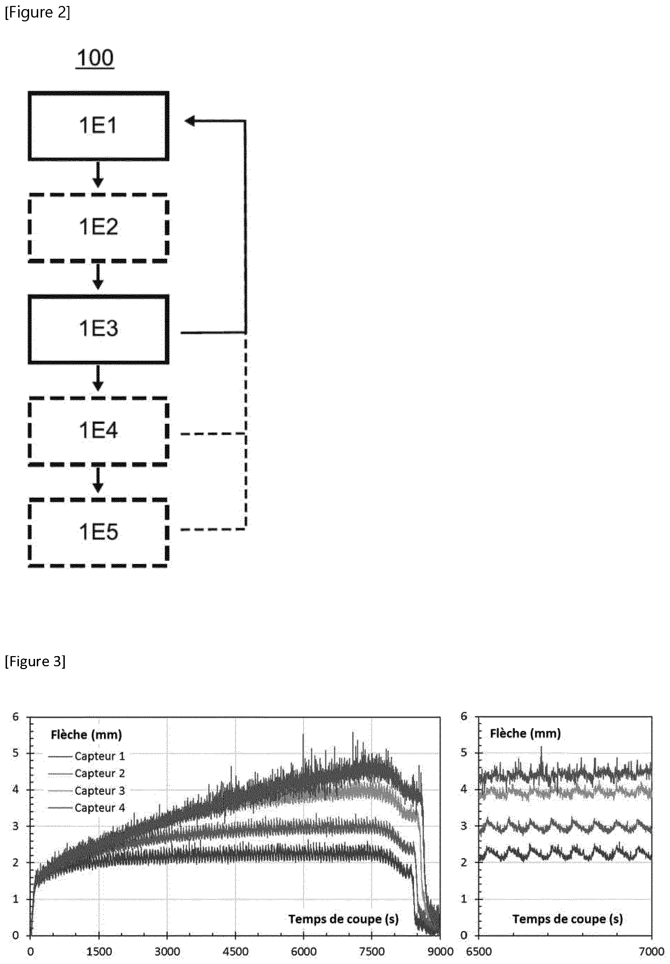

Un premier aspect de l'invention illustré aux figures 1 et 2 concerne un procédé 100 de suivi de la coupe en fonction de la flèche FL d'au moins un fil FI de coupe, de préférence une pluralité de fils FL de coupe, à l'aide d'au moins un capteur CA de flèche, de préférence une pluralité de capteurs CA. Dans les faits, le fil FI abrasif est en général enroulé plusieurs centaines de fois autour de guide-fils cylindriques GF rotatifs rainurés qui déterminent l'écartement entre les passes, pour former une nappe. En outre, plusieurs capteurs CA sont disposés le long de cette nappe afin de mesurer la flèche FL en différents points de ladite nappe. Aussi, dans la suite de la description, la nappe qui est en fait constituée d'un fil FI sera considérée comme une pluralité de fils FI, chaque fil FI de la pluralité de fils FI correspondant alors à un fil FI de la nappe (voir figure 1).

Comme illustré à la figure 1, lors de la découpe, il est possible d'associer à chaque capteur CA une flèche FL, ladite flèche FL pouvant être caractérisée par un angle $\alpha$. On remarquera que cet angle $\alpha$ permet également de remonter à la flèche exprimée en unité de longueur, la relation liant la « flèche angulaire » à la « flèche » étant fonction de la géométrie de l'appareil de coupe. De manière générale, un capteur CA mesure la flèche FL de plusieurs fils FI. Cependant, le procédé 100 selon un premier aspect de l'invention peut également être mis en oeuvre avec un capteur CA mesurant la flèche d'un seul fil FI de la nappe. On entend par capteur CA de flèche, un capteur CA qui, à partir de la mesure d'une ou plusieurs grandeurs physiques, permet de connaître la flèche FL d'un fil FI ou d'une pluralité de fils FI. Dans la suite, à de fins d'illustration et sauf précision contraire, la flèche FL fera référence à la « flèche angulaire » et lorsqu'une valeur de cette dernière sera donnée (en degré donc), une valeur de la « flèche » (en mm) sera en général donnée. La valeur de cette « flèche » (en mm) est calculée pour une scie industrielle de découpe de wafers de silicium Meyer Burger DW288 P6 série 3 équipée de 2 guide-fils GF de 200 mm de diamètre installés avec un entraxe de 390 mm.

De plus, tous les exemples donnés à des fins d'illustration ont été obtenus lors de coupes réalisées avec un fil FI diamanté de diamètre d'âme 80 $\mu$m type ASAHI 80HT 8-16 HC+. Pour cela, huit capteurs CA de proximité inductifs (sans contact) quasi-noyables ont été installés dans la chambre de coupe, entre le guide-fil GL amont (côté entrée du fil) et la brique BR de silicium. Les capteurs CA, distants d'environ 24,5 mm de la génératrice supérieure du guide-fil GF, ont été espacés d'environ 60 mm, afin de réaliser plusieurs mesures quasi locales de flèche FL. De manière générale, le nombre de capteurs CA effectivement utilisés (c'est-à-dire participant à la mesure de la flèche FL) dépend de la largeur de la nappe de fil FI, elle-même liée à longueur de la brique BR de silicium découpée, cette dernière variant en général entre 100 et 500 mm environ. Ces capteurs CA délivrent des signaux électriques 0-10 volts (analogiques) qui sont enregistrés par un système d'acquisition avec une fréquence typique de 2 Hz.

Afin de déterminer cette flèche FL, le procédé 100 selon un premier aspect de l'invention comprend, pour chaque capteur CA, une étape 1E1 de mesure de la flèche $\alpha$ du fil FI. Cette mesure peut nécessiter la calibration des capteurs CA. De manière générale, lorsque la coupe est effectuée sur une brique, les capteurs CA sont calibrés in situ en appuyant la brique BR sur la nappe de fil FI et en la faisant descendre par paliers, typiquement entre 0 et 10 mm. Ainsi, bien que la mesure de la flèche ne soit pas réalisée au niveau de la brique BR, cette calibration reflète bien la déflexion maximale du fil FI (et donc la valeur de la flèche). Les courbes de calibration flèche - tension de chaque capteur actif sont ensuite ajustées par des polynômes. Cette procédure de calibration des capteurs CA est cependant bien connue de l'homme du métier et ne sera donc pas détaillée.

L'évolution de la flèche FL au cours de la coupe est illustré à la figure 3 qui montre l'évolution de la flèche mesurée dans quatre zones équidistantes au moyen de quatre capteurs CA distincts pendant la coupe d'une brique BR de silicium mono-like de longueur 260 mm, le fil FI de coupe formant une nappe de 227 mm. Dans cet exemple, le premier capteur CA (capteur 1) est implanté du côté de l'entrée du fil FI neuf dans la nappe alors que le quatrième capteur CA (capteur 4) se situe à proximité de la sortie du fil FI usagé. L'usure de celui-ci se traduit par une augmentation de sa flèche FL, qui passe de 2 mm à moins de 5 mm au maximum, vers 7500 secondes, soit environ 80% de la coupe. Une valeur supérieure à 8 mm, correspondant à un angle $\alpha$ de 2,3° par rapport à la position au repos, serait critique pour la tenue du fil FI (risque de rupture), surtout si elle est atteinte avant cette durée de sciage. Les courbes présentent des fluctuations à basse fréquence liées au mouvement d'aller-retour du fil FI (voir détail de droite de la figure 3).

Pour réduire ce bruit, les mesures de la flèche FL peuvent être traitées numériquement afin de faciliter par la suite la détermination de la dérivée temporelle de la flèche FL. Pour cela, dans un mode de réalisation, le procédé 100 selon un premier aspect de l'invention comprend, pour chaque capteur CA, une étape 1E2 de traitement de la mesure de la flèche FL, la dérivée temporelle de la flèche FL étant déterminée à partir de la mesure ainsi traitée.

Les inventeurs ont constaté que la prise en compte de la fréquence d'aller-retour du fil FI dans le traitement numérique permettait d'améliorer la réduction du bruit. Aussi, dans un mode de réalisation l'étape 1E2 de traitement de la mesure de la flèche FL comprend un lissage de la mesure, le mouvement d'aller-retour du fil FI étant effectué à une fréquence déterminée et le lissage de la mesure issue du capteur CA étant effectuée en fonction de la fréquence du mouvement d'aller-retour.

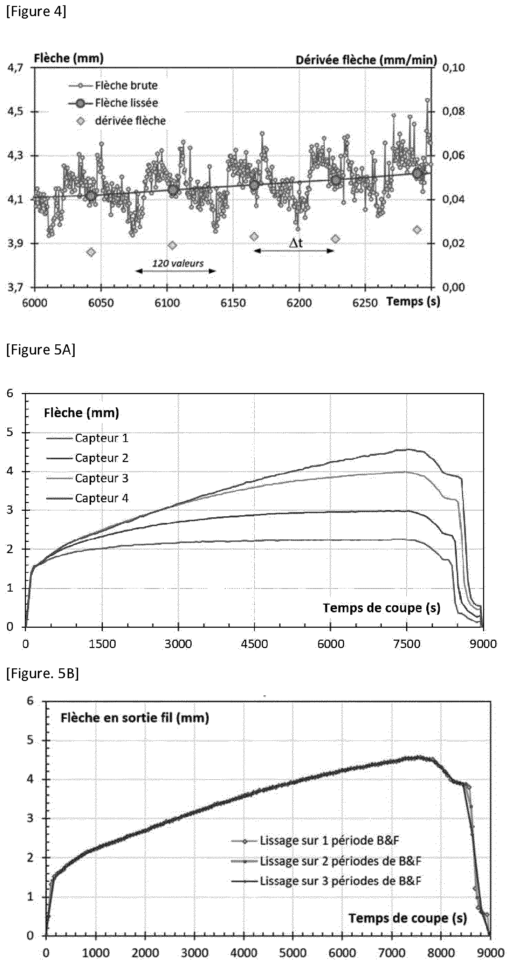

Le principe d'un tel lissage est présenté à la figure 4 et sa mise en oeuvre est illustrée à la figure 5A et à la figure 5B. Dans l'exemple illustré sur ces figures, le lissage de la mesure est effectué en calculant la moyenne sur une période d'aller-retour (c'est-à-dire le temps séparant le début d'un aller de fil avec le début de l'aller suivant - ici 60 secondes, soit 120 points à la fréquence d'acquisition du système de 2 Hz), chaque valeur moyenne ainsi obtenue étant affectée au temps moyen de l'aller-retour considéré. La figure 4 représente une mesure de la flèche FL brute, de la mesure lissée de cette flèche FL ainsi que la dérivée de la mesure lissée de cette flèche FL en fonction du temps, la période d'aller-retour étant notée $\Delta t$ sur cette figure. La mesure lissée de la flèche FL est représentée pour plusieurs capteurs CA à la figure 5A. Dans cet exemple, le lissage est effectué en prenant en considération une période d'aller-retour et présente encore quelques fluctuations qui ne compromettent cependant pas le suivi désiré. Il est toutefois possible de diminuer encore ces fluctuations en prenant en compte un nombre plus grand de périodes d'aller-retour, la moyenne étant alors calculée sur le nombre de périodes considérées. La figures 5B (où B&F - pour Back & Forth - fait référence aux allers-retours) illustre l'influence du nombre de périodes considérées pour le lissage sur la mesure de la flèche FL.

Le suivi en temps réel de la flèche FL, de préférence en plusieurs points de la nappe, permet d'évaluer la difficulté de sciage et de déclencher une intervention des opérateurs lorsque certaines limites sont atteintes. Cependant, ce seul paramètre ne permet pas une anticipation suffisante dans tous les cas. Afin d'améliorer l'anticipation dans le suivi de la flèche FL, les inventeurs ont mis en évidence que la dérivée temporelle première de la flèche FL, en renseignant sur la cinétique d'évolution de cette dernière, permettait d'appréhender au plus tôt les éventuels problèmes, en se basant sur des critères appropriés. Afin d'exploiter cette propriété, le procédé 100 de suivi selon un premier aspect de l'invention comprend également, pour chaque capteur CA, une étape 1E3 de détermination de la dérivée temporelle première $\dot{\alpha}$ de ladite flèche $\alpha$.

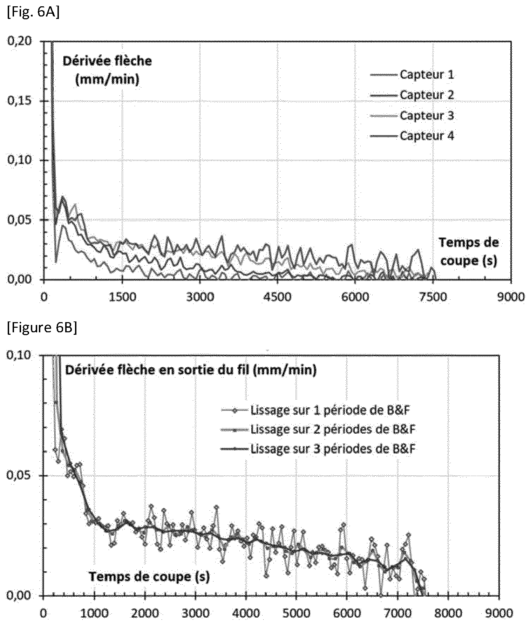

Ainsi, une fois le lissage de la mesure de la flèche FL effectué, la dérivée temporelle première de la flèche FL est calculée comme le montre la figure 6A illustrant l'évolution cette dérivée temporelle en fonction du temps pour les quatre capteurs CA évoqués plus haut. La figure 6B (où B&F - pour Back & Forth - fait référence aux allers-retours) illustre quant à elle l'influence du nombre de périodes considérées pour le lissage de la mesure sur la dérivée temporelle première de la flèche FL.

Les étapes qui viennent d'être décrites 1E1, 1E3 sont réitérées à intervalles réguliers de sorte à assurer un suivi de la coupe, ce suivi se faisant non seulement à partir de la valeur de la flèche FL, mais également à partir de la dérivée temporelle première de cette dernière.

Dans un mode de réalisation, le procédé 100 comprend une étape 1E4 d'établissement d'un statut de la coupe en fonction de la flèche $\alpha$ du fil FI et de la dérivée temporelle première $\dot\alpha$ de la flèche FL. On comprendra ici que ce statut sera mis à jour à chaque itération de l'étape 1E1 étape de mesure de la flèche $\alpha$ du fil FI, de l'étape 1E2 de traitement de la mesure de la flèche FL si celle-ci est mise en oeuvre et de l'étape 1E3 de détermination de la dérivée temporelle première $\dot\alpha$ de la flèche $\alpha$.

Dans un mode de réalisation, le statut de la coupe est considéré comme « nominal » si, pour chaque capteur CA, $\alpha$ est inférieur à un premier seuil $S_{1*}^{\alpha}$ **et** si, pour chaque capteur CA, $\dot\alpha$ est inférieur à un deuxième seuil $S_{2*}^{\dot\alpha}$ **alors que** la coupe est avancée à plus de 15%. Par exemple, le premier seuil $S_{1*}^{\alpha}$ pourra être choisi comme égal à 2,3° (soit 8 mm) tandis que le deuxième seuil $S_{2*}^{\dot\alpha}$ pourra être choisi comme étant égal à 0,014°/minute (environ 0,05 mm/minute). De manière plus générale, une coupe qui n'a ni le statut de « à risque », ni le statut de « dangereux » tels que décrits dans la suite est considérée comme étant dans un statut « nominal ».

Dans un mode de réalisation, le statut de la coupe est considéré comme « à risque » si, pour un et un seul capteur CA, $\alpha$ est supérieur au premier seuil $S_{1*}^{\alpha}$, **et** si, pour un nombre de capteurs CA prédéterminé, $\dot\alpha$ est supérieur au deuxième seuil $S_{2*}^{\dot\alpha}$ et inférieur à un troisième seuil $S_{3*}^{\dot\alpha}$ **alors que** la coupe est avancée à plus de 15%. Par exemple, le premier seuil $S_{1*}^{\alpha}$ pourra être choisi comme étant égal à 2,3° (8 mm), le deuxième seuil $S_{2*}^{\dot\alpha}$ pourra être choisi comme étant égal à 0,014°/minute (environ 0,05 mm/minute) et le troisième seuil $S_{3*}^{\dot\alpha}$ pourra être choisi comme étant égal à 0,023°/minute (environ 0,08 mm/minute). Dans un mode de réalisation, lorsque la coupe concerne une brique BR, le nombre de capteurs CA prédéterminé est égal au nombre de capteurs CA couvrant une zone de la nappe correspondant à la moitié de la longueur de la brique BR.

Dans un mode de réalisation, le statut de la coupe est considéré comme « dangereux » lorsque, pour au moins deux capteurs CA, $\alpha$ est supérieur au premier seuil $S_{1*}^{\alpha}$ **ou** lorsque, pour au moins deux capteurs CA, $\dot\alpha$ est supérieur au troisième seuil $S_{3*}^{\dot\alpha}$ **alors que** la coupe est avancée à plus de 10%. Par exemple, le premier seuil $S_{1*}^{\alpha}$ pourra être choisi comme étant égal à 2,3° (8 mm) et le troisième seuil $S_{3*}^{\dot\alpha}$ pourra être choisi comme étant égal à 0,023°/minute (0,08 mm/minute). Ce statut permet de faciliter le suivi en identifiant par exemple les régimes de fonctionnement « nominaux », « à risque » ou bien encore « dangereux ».

Si une coupe venait à pouvoir être classé dans plusieurs statuts, alors le statut « dangereux » prévaut sur les statuts « à risque » et « nominal », et le statut « à risque » prévaut sur le statut « nominal ».

Dans un mode de réalisation, le procédé 100 selon un premier aspect de l'invention comprend une étape 1E5 de correction des paramètres de la coupe et/ou une étape d'émission d'une alerte en fonction du statut de la coupe ou de la valeur de la flèche FL et de sa dérivée première lorsque le procédé ne comprend pas d'étape de détermination d'un statut de la coupe. Par exemple, les paramètres de coupe peuvent être modifiés lorsque le statut de la coupe change. On peut par exemple envisager que le passage d'un statut « nominal » à un statut « à risque » entraine une augmentation de la vitesse de défilement du fil FI et/ou un ralentissement de la vitesse de la brique BR. De même, le passage d'un statut de « à risque » à « dangereux » peut entrainer l'arrêt de la coupe. L'émission d'une alerte peut être effectuée selon des critères similaires. L'alerte peut par exemple prendre la forme d'une alerte sonore et/ou lumineuse ou bien encore la forme d'un message envoyé sous forme électronique, par exemple par SMS ou courriel. Elle vise notamment à prévenir l'opérateur qu'une intervention ou une attention accrue est nécessaire.

Dans un mode de réalisation, le procédé 100 selon un premier aspect de l'invention comprend une étape de détermination du rapport entre la flèche FL et l'avancement de la coupe ou du temps de coupe (c'est-à-dire le temps

$$\frac{\text{Flèche(° ou mm)}}{\text{Avancement de la coupe ou temps de coupe (\% ou seconde)}}$$

écoulé depuis le début de la coupe), c'est à dire , le suivi de la coupe étant également fonction de la valeur dudit rapport. Ainsi, en plus de la dérivée temporelle de la flèche, le suivi de la coupe peut également prendre en compte le rapport entre la flèche et l'avancement de la coupe ou le temps de coupe, permettant la détection d'éventuelles anomalies qui n'apparaitraient pas dans la dérivée temporelle.

Dans un mode de réalisation, la coupe est considérée comme étant dans un statut nominal si, pour tous les capteurs CA, l'avancement de la coupe étant supérieur ou égal à 15%, le rapport entre la flèche FL et l'avancement de la coupe est inférieur à 0,0575°/% (c'est-à-dire 0,2 mm/%). Dans un mode de réalisation, la coupe est considérée comme étant dans un statut nominal si, pour tous les capteurs, l'avancement de la coupe étant supérieur ou égal à 40%, le rapport entre la flèche FL et l'avancement de la coupe est inférieur à 0,02875°/% (c'est-à-dire 0,1 mm/%).

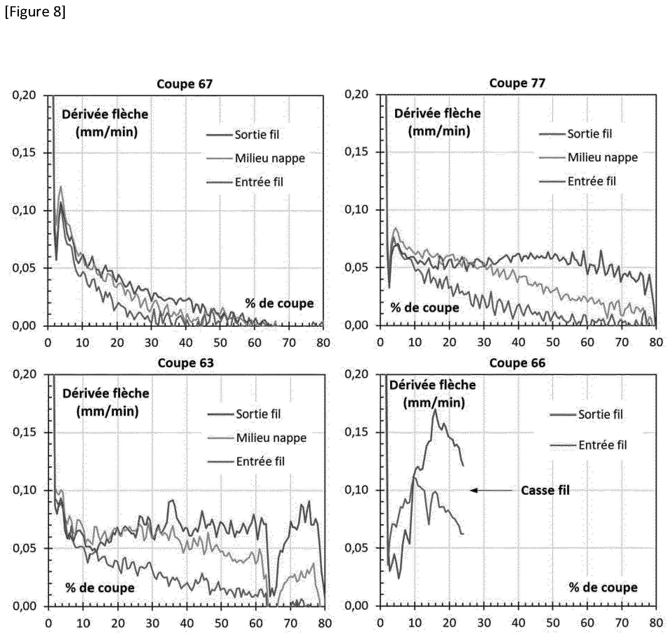

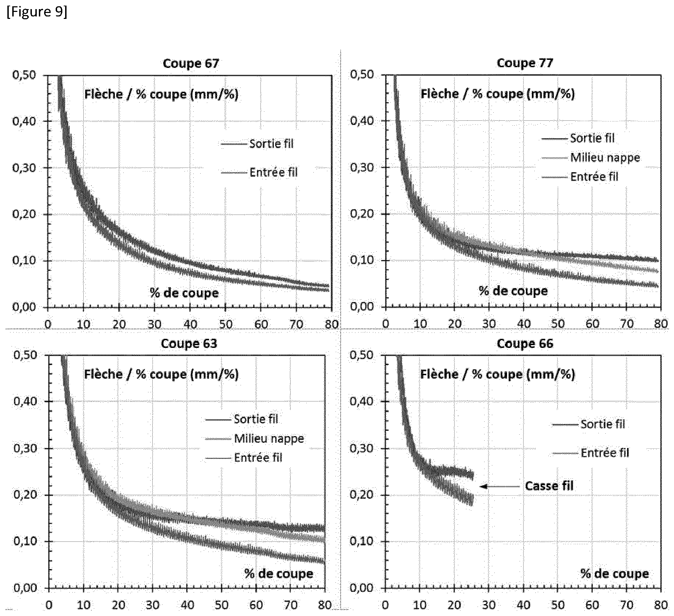

Un exemple de réalisation d'un procédé 100 selon un premier aspect de l'invention est illustré aux figures 7, 8 et 9, la figure 7 illustrant l'évolution de la flèche FL, la figure 8 illustrant sa dérivée temporelle première et la figure 9 illustrant le rapport entre la flèche et l'avancement de la coupe en fonction de l'avancement de la coupe. Les conditions de sciage et de mesure sont résumées dans le tableau 1 ci-dessous. Dans cet exemple de réalisation, on choisira le premier seuil $S_{1*}^{\alpha}$ comme étant égal à 2,3° (8 mm), le deuxième seuil $S_{2*}^{\dot{\alpha}}$ comme étant égal à 0,014°/minute (environ 0,05 mm/minute) et le troisième seuil $S_{3*}^{\dot{\alpha}}$ comme étant égal à 0,023°/minute (environ 0,08 mm/minute).

[Tableau 1]

| N° coupe | Matériau | Longueur brique (mm) | Largeur nappe de fil (mm) | Consommation fil (m/ wafer) | Durée coupe (min) | Nombre capteurs |
|---|---|---|---|---|---|---|
| 63 | Mono-like | 270 | 230 | 1,0 | 165 | 4 |
| 66 | Multicristallin | 178 | 120 | 2,0 | 43 | 2 |
| 67 | Mono-like | 270 | 220 | 2,4 | 156 | 3 |
| 76 | Mono-like | 260 | 227 | 1,5 | 153 | 4 |
| 77 | Mono-like | 195 | 190 | 1,5 | 157 | 3 |

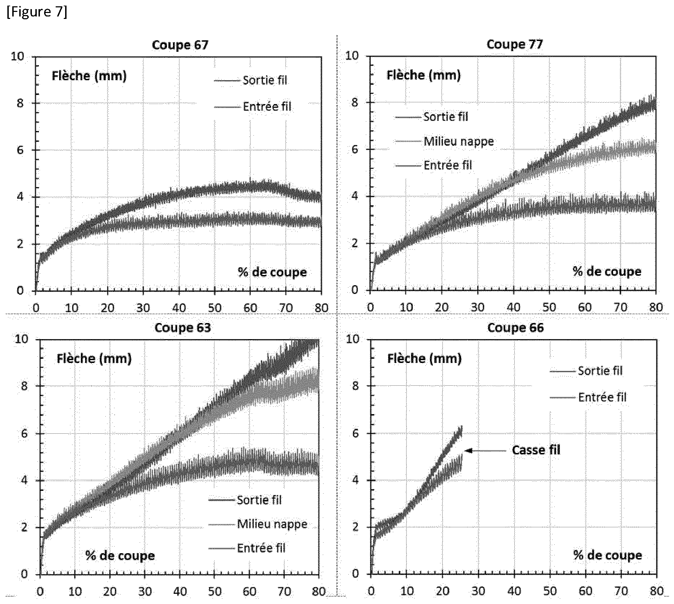

Les coupes du tableau 1 sont ordonnées par difficulté croissante. La difficulté associée à une coupe peut être caractérisée par plusieurs paramètres, notamment les caractéristiques du matériau scié (en particulier la présence de précipités de $Si_3N_4$ ou SiC), le renouvellement de fil (consommation par wafer), etc. La coupe n°67 représentée en haut à gauche de la figure 7 et de la figure 8 s'est déroulée sans problème, autrement dit, le statut de la coupe est resté « nominal » tout le long de la coupe. Ceci ressort des valeurs de flèche FL faibles, inférieures à 5 mm pour l'ensemble des capteurs CA, et une dérivée temporelle première inférieure à 0,05 mm/minute au-delà de 15% de la coupe, quel que soit le capteur CA considéré.

Les coupes n° 77 et 63 respectivement représentées en haut à droite et en bas à gauche de la figure 7 et de la figure 8 se sont déroulées dans des conditions plus difficiles bien qu'elles aient pu être menées à terme.

Dans le cas de la coupe n° 77, plusieurs allers-retours du fil FI ont dû être effectués après la fin de la coupe afin de rattraper l'importante flèche FL prise par le fil FI en sortie (sensiblement égale à 8 mm). Outre l'allongement des opérations, néfaste à la productivité en situation industrielle, une telle reprise engendre un risque de chute des wafers en entrée, leur support étant alors fragilisé par le passage prolongé du fil FI abrasif. La difficulté de la coupe peut notamment se lire sur l'évolution de la dérivée temporelle première de la flèche FL à la figure 8. Sur cette dernière, passé le seuil des 15% de coupe, la dérivée temporelle première de la flèche FL au niveau de la sortie de fil FI est supérieure à 0,05 mm/min. Autrement dit, le statut de la coupe aurait été identifié comme étant « à risque » par un procédé 100 selon un premier aspect de l'invention.

Dans le cas de la coupe n° 63, la flèche FL maximale ayant dépassé 8 mm à environ 60% de la coupe, la vitesse de descente de la brique BR a dû être ralentie de 10% à deux reprises. Suivi par un procédé 100 selon un premier aspect de l'invention le statut de la coupe serait passé de « nominal » à « à risque » entraînant par exemple une modification de la descente de la brique BR (il ne s'agit que d'un exemple de rétroaction possible).

Dans les deux cas, la dérivée temporelle première de la flèche en sortie était globalement légèrement croissante et se maintenait longuement au-dessus de 0,05 mm/min. Par contre, les évolutions restaient globalement décroissantes pour tous les capteurs CA situés en amont, mais de manière moins marquée pour la coupe n° 63. Autrement dit, lors d'un suivi à l'aide d'un procédé 100 selon un premier aspect de l'invention, la coupe n'aurait pas atteint le statut « dangereux ».

Contrairement aux autres, la coupe n° 66 illustrée en bas à droite des figures 7 et 8 s'est terminée de manière prématurée, avec une rupture du fil FI vers 26% d'avancement. Dans ce cas, bien qu'élevées, les valeurs de flèche FL étaient restées dans des limites a priori acceptables. Une condition sur ce seul paramètre n'aurait donc pas permis de détecter un risque de rupture du fils. En revanche, très rapidement, la dérivée première de la flèche FL dépassait nettement les 0,05 mm/minute. Ainsi, dès 10% de la coupe, un procédé 100 selon l'invention aurait identifié la coupe comme ayant un statut « dangereux » et aurait pu alerter les opérateurs et/ou modifier les paramètres de coupe. Dans ce cas, particulièrement critique (en raison des caractéristiques du matériau), il est probable que seule une interruption de la coupe aurait permis de préserver le fil.

La figure 9 montre qu'en l'absence de difficulté, le rapport de la flèche (FL) sur l'avancement de la coupe tend vers 0,05 mm/% (cf. coupe 67). Dans le cas contraire, la valeur de ce rapport peut dépasser 0,10 mm/% (au moins pour certains capteurs - cf. coupes 77 et 63) ou présenter une inflexion marquée (capteur de sortie lors de la coupe 66).

Comme on vient de le voir, grâce à un procédé selon un premier aspect de l'invention, il est possible d'effectuer un suivi de la coupe à partir de la flèche FL et de sa dérivée temporelle première. Cependant, il peut parfois être utile de prendre également en compte la dérivée temporelle seconde de la flèche FL. Pour cela, dans un mode de réalisation, le procédé 100 selon un premier aspect de l'invention comprend également une étape de détermination de la dérivée temporelle seconde $\ddot{\alpha}$ de ladite flèche $\alpha$. Dans ce mode de réalisation, lorsque le lissage est effectué en fonction du mouvement aller-retour du fil FL, il est préférable d'effectuer une ou plusieurs coupes de test afin de s'assurer que le nombre de périodes d'aller-retour prises en compte pour le lissage de la mesure est suffisant pour éviter de faux positifs induits par des fluctuations du signe de la dérivée temporelle seconde de la flèche.

Dans un mode de réalisation, lors de l'étape 1E4 d'établissement d'un statut de la coupe, la dérivée temporelle seconde $\ddot{\alpha}$ de la flèche $\alpha$ est également prise en compte. Ce statut sera mis à jour à chaque itération de l'étape 1E1 étape de mesure de la flèche $\alpha$ du fil FI, de l'étape 1E2 de traitement de la mesure de la flèche FL ci cette dernière est mise en oeuvre et de l'étape 1E3 de détermination de la dérivée temporelle première $\dot{\alpha}$ et, dans ce mode de réalisation, de la dérivée temporelle seconde $\ddot{\alpha}$ de ladite flèche $\alpha$.

Dans un mode de réalisation, le statut de la coupe est considéré comme « dangereux » lorsque, pour au moins deux capteurs CA, $\alpha$ est supérieur à un premier seuil $S_1^{\alpha}$ **ou** lorsque, pour au moins deux capteurs CA, $\ddot{\alpha}$ est supérieur à un deuxième seuil $S_2^{\ddot{\alpha}}$ **et** si, pour ces deux capteurs CA, $\dot{\alpha}$ est supérieur à un cinquième seuil $S_5^{\dot{\alpha}}$. Il est important de noter que la phrase précédente doit être comprise comme « A ou (B et C) » avec A «pour au moins deux capteurs CA, $\alpha$ est supérieur à un premier seuil $S_1^{\alpha}$ », B «pour au moins deux capteurs CA, $\ddot{\alpha}$ est supérieur à un deuxième seuil $S_2^{\ddot{\alpha}}$ » et C « pour ces deux capteurs CA, $\dot{\alpha}$ est supérieur à un cinquième seuil $S_5^{\dot{\alpha}}$ ». Par exemple, le premier seuil $S_1^{\alpha}$ pourra être choisi comme étant égal à 2,3° (8 mm) et le cinquième seuil $S_5^{\dot{\alpha}}$ pourra être choisi comme étant égale à 0.023°/minute (0,08 mm/minute). Ce statut permet de faciliter le suivi en identifiant par exemple les régimes de fonctionnement « nominaux », « à risque » ou bien encore « dangereux ».

Dans un mode de réalisation, le statut de la coupe est considéré comme « nominal » si, pour chaque capteur CA, $\alpha$ est inférieur à un premier seuil $S_1^{\alpha}$ **et** si, pour chaque capteur CA, $\ddot{\alpha}$ est inférieur à un deuxième seuil $S_2^{\ddot{\alpha}}$. Par exemple, le premier seuil $S_1^{\alpha}$ pourra être choisi comme égal à 2,3° (soit 8 mm) tandis que le deuxième seuil $S_2^{\ddot{\alpha}}$ pourra être choisi comme étant égal à 0 (autrement dit, la dérivée temporelle seconde $\ddot{\alpha}$ est négative, la dérivée temporelle première $\dot{\alpha}$ étant alors décroissante).

Dans un mode de réalisation, le statut de la coupe est considéré comme « à risque » si, pour un et un seul capteur CA, $\alpha$ est supérieur à un premier seuil $S_1^{\alpha}$, **et** si, pour un nombre de capteurs CA prédéterminé, $\ddot{\alpha}$ est inférieur à un troisième seuil $S_3^{\ddot{\alpha}}$, **et,** lorsque la coupe est avancée à plus de 15%, si, pour au moins un capteur CA, $\ddot{\alpha}$ est supérieur à un quatrième seuil $S_4^{\ddot{\alpha}}$. On notera de ce qui précède que le critère concernant la dérivée première $\dot{\alpha}$ de la flèche $\alpha$ n'est examiné que si la coupe est avancée d'au moins 15%. Par exemple, le premier seuil $S_1^{\alpha}$ pourra être choisi comme étant égal à 2,3° (8 mm), le troisième seuil $S_3^{\ddot{\alpha}}$ comme étant égal à 0,43.10⁻³°/minute² (environ 1,5.10⁻³

mm/minute$^2$), et le quatrième seuil $S_4^{\ddot{\alpha}}$ comme étant égal à 0,014°/minute (environ 0,05 mm/minute). Dans un mode de réalisation, lorsque la coupe concerne une brique BR, le nombre de capteurs CA prédéterminé est égal au nombre de capteurs CA couvrant une zone de la nappe correspondant à la moitié de la longueur de la brique BR.

Afin de mettre en oeuvre un procédé 100 selon un premier aspect de l'invention, un troisième aspect de l'invention concerne un dispositif comprenant les moyens pour mettre en oeuvre ledit procédé 100. Dans un mode de réalisation, le dispositif comprend au moins un capteur CA de flèche FL, de préférence une pluralité de capteurs CA de flèche FL. Dans un mode de réalisation, le dispositif comprend un moyen de calcul (par exemple un processeur) associé à une mémoire (par exemple une mémoire RAM), la mémoire étant configurée pour stocker les instructions ainsi que les données nécessaires à la mise en oeuvre d'un procédé 100, 200 selon un premier aspect de l'invention ou un deuxième aspect de l'invention. Dans un mode de réalisation, le dispositif comprend en outre des moyens pour échanger des données entre le ou les capteurs CA et le moyen de calcul de sorte que ce dernier puisse acquérir et traiter les mesures de flèche FL issues du ou des capteurs CA.

## Revendications

1. Procédé (100) de suivi de la coupe, la coupe étant effectuée avec un mouvement aller-retour du fil (FI) de coupe, le mouvement aller-retour du fil (FI) étant effectué à une fréquence déterminée, le suivi étant effectué en fonction de la flèche (FL) d'au moins un fil (FI) de coupe à l'aide d'au moins un capteur (CA), le fil (FI) présentant une flèche (FL) lors de la coupe, ladite flèche (FL) étant **caractérisée par** un angle $\alpha$, le procédé (100) comprenant, pour chaque capteur (CA) :

   - une étape (1E1) de mesure de la flèche $\alpha$ du fil ;
   - une étape (1E2) de traitement de la mesure de la flèche (FL) comprenant le lissage de la mesure issue du capteur (CA), ledit lissage étant effectué en fonction de la fréquence du mouvement aller-retour du fil (FI) ;
   - une étape (1E3) de détermination de la dérivée temporelle première $\dot{\alpha}$ de la flèche $\alpha$ à partir de la mesure lissée ;

   lesdites étapes (1E1, 1E2, 1E3) étant réitérées à intervalles réguliers de sorte à assurer un suivi de la coupe.

2. Procédé (100) selon la revendication précédente **caractérisé en ce qu'**il comprend une étape (1E4) d'établissement d'un statut de la coupe en fonction de la flèche $\alpha$ du fil et de la dérivée temporelle première $\dot{\alpha}$ de ladite flèche $\alpha$ mesurée par chaque capteur.

3. Procédé (100) selon la revendication précédente **caractérisé en ce qu'**il comprend une étape (1E5) de correction des paramètres de la coupe et/ou une étape d'émission d'une alerte en fonction du statut de la coupe ou de la valeur de la flèche FL et de sa dérivée temporelle première.

4. Procédé (100) selon l'une des deux revendications précédentes **caractérisé en ce que** le statut de la coupe est « nominal » si, pour chaque capteur CA, $\alpha$ est inférieur à un premier seuil $S_{1*}^{\alpha}$ et si, pour chaque capteur CA, $\dot{\alpha}$ est inférieur à un deuxième seuil $S_{2*}^{\dot{\alpha}}$ lorsque la coupe est avancée à plus de 15%.

5. Procédé (100) selon l'une des trois revendications précédentes **caractérisé en ce que** le statut de la coupe est « à risque » si, pour un et un seul capteur CA, $\alpha$ est supérieur à un premier seuil $S_{1*}^{\alpha}$ , et si, pour un nombre de capteurs CA prédéterminé, $\dot{\alpha}$ est supérieur à un deuxième seuil $S_{2*}^{\dot{\alpha}}$ et inférieur à un troisième seuil $S_{3*}^{\dot{\alpha}}$ lorsque la coupe est avancée à plus de 15%.

6. Procédé (100) selon l'une des quatre revendications précédentes **caractérisé en ce que** le statut de la coupe est « dangereux » si, pour au moins deux capteurs CA, $\alpha$ est supérieur à un premier seuil $S_{1*}^{\alpha}$ ou lorsque, pour au moins deux capteurs CA, $\dot{\alpha}$ est supérieur à un troisième seuil $S_{3*}^{\dot{\alpha}}$ alors que la coupe est avancée à plus de 10%

7. Procédé (100) selon l'une des revendications précédentes **caractérisé en ce qu'**il comprend une étape de détermination du rapport entre la flèche (FL) et l'avancement de la coupe ou le temps de coupe, le suivi de la coupe étant également fonction de la valeur dudit rapport.

8. Procédé (200) de suivi de la coupe en fonction de la flèche (FL) d'au moins deux fils (FI) de coupe à l'aide d'au moins deux capteurs (CA), le fil (FI) présentant une flèche (FL) lors de la coupe, ladite flèche (FL) pouvant être **caractérisée par** un angle $\alpha$, le procédé (100) de suivi étant **caractérisé en ce qu'**il comprend, pour chaque capteur (CA) :

- une étape (2E1) de mesure de la flèche $\alpha$ du fil ;
- une étape (2E3) de détermination de la dérivée temporelle première $\dot{\alpha}$ et seconde $\ddot{\alpha}$ de ladite flèche $\alpha$ ;

le procédé comprenant en outre une étape (2E4) d'établissement d'un statut de la coupe en fonction de la flèche $\alpha$ du fil (FI), de la dérivée temporelle première $\dot{\alpha}$ et de la dérivée temporelle seconde $\ddot{\alpha}$ de ladite flèche $\alpha$ mesurée par chaque capteur (CA), le statut de la coupe étant « dangereux » lorsque, pour au moins deux capteurs (CA), $\alpha$ est supérieur au premier seuil $S_1^{\alpha}$ ou lorsque, pour au moins deux capteurs (CA), $\ddot{\alpha}$ est supérieur au deuxième seuil $S_2^{\ddot{\alpha}}$ et si, pour ces deux capteurs (CA), $\dot{\alpha}$ est supérieur à un cinquième seuil $S_5^{\dot{\alpha}}$, lesdites étapes (2E1, 2E3, 2E4) étant réitérées à intervalles réguliers de sorte à assurer un suivi de la coupe.

9. Procédé (200) selon la revendication précédente **caractérisé en ce qu'**il comprend, pour chaque capteur (CA), après l'étape (2E1) de mesure de la flèche $\alpha$ et avant l'étape (2E3) de détermination de la dérivée temporelle première $\dot{\alpha}$ et seconde $\ddot{\alpha}$ de ladite flèche $\alpha$, une étape (2E2) de traitement de la mesure de la flèche (FL).

10. Procédé (200) selon la revendication précédente **caractérisé en ce que** la coupe dont le suivi est assuré est effectuée avec un mouvement aller-retour du fil (FI) de coupe, le mouvement aller-retour du fil (FI) étant effectué à une fréquence déterminée et **en ce que** l'étape (2E2) de traitement de la mesure de la flèche (FL) comprend une sous-étape de lissage de la mesure issue des capteurs, ledit lissage étant effectué en fonction de la fréquence du mouvement aller-retour du fil (FL).

11. Procédé (200) selon l'une des revendications 8 à 10 **caractérisé en ce qu'**il comprend une étape (2E5) de correction des paramètres de la coupe et/ou une étape d'émission d'une alerte en fonction du statut de la coupe.

12. Procédé selon l'une des revendications 8 à 11 **caractérisé en ce que** le statut de la coupe est « à risque » si, pour un et un seul capteur (CA), $\alpha$ est supérieur au premier seuil $S_1^{\alpha}$, et si, pour un nombre de capteurs (CA) prédé-terminé, $\ddot{\alpha}$ est inférieur à un troisième seuil $S_3^{\ddot{\alpha}}$, et, lorsque la coupe est avancée au-delà d'un seuil d'avancement prédéterminé, si, pour au moins un capteur (CA), $\dot{\alpha}$ est supérieur à un quatrième seuil $S_4^{\dot{\alpha}}$.

13. Procédé selon l'une des revendications 8 à 12 **caractérisé en ce qu'**il comprend une étape de détermination du rapport entre la flèche (FL) et l'avancement de la coupe ou le temps de coupe, le suivi de la coupe étant également fonction de la valeur dudit rapport.

14. Dispositif de suivi de la coupe comprenant des moyens pour mettre en oeuvre un procédé selon l'une des revendications précédentes.

15. Programme d'ordinateur comprenant des instructions qui conduisent le dispositif selon la revendication précédente à exécuter les étapes du procédé selon l'une des revendications 1 à 13.

16. Support lisible par ordinateur, sur lequel est enregistré le programme d'ordinateur selon la revendication précédente.

**Patentansprüche**

1. Schnittverfolgungsverfahren (100), wobei der Schnitt mit einer Hin- und Rückbewegung des Schneidfadens (FI) durchgeführt wird, die Hin- und Rückbewegung des Fadens (FI) mit einer bestimmten Frequenz durchgeführt wird, die Verfolgung in Abhängigkeit des Durchhangs (FL) mindestens eines Schneidfadens (FI) mit Hilfe mindestens eines Sensors (CA) durchgeführt wird, der Faden (FI) beim Schneiden einen Durchhang (FL) aufweist und dieser Durchhang (FL) durch einen Winkel $\alpha$ gekennzeichnet ist, wobei das Verfahren (100) für jeden Sensor (CA) umfasst:

- einen Messschritt (1E1) des Durchhangs $\alpha$ des Fadens;
- einen Verarbeitungsschritt (1E2) des Messwerts des Durchhangs (FL), der die Glättung der von dem Sensor (CA) stammenden Messung umfasst, wobei diese Glättung in Abhängigkeit der Frequenz der Hin- und Rückbewegung des Fadens (FI) durchgeführt wird;
- einen Bestimmungsschritt (1E3) der ersten zeitlichen Ableitung $\dot\alpha$ des Durchhangs $\alpha$ ausgehend von der geglätteten Messung;

wobei die Schritte (1E1, 1E2, 1E3) in regelmäßigen Abständen wiederholt werden, so dass ein Verfolgen des Schnitts gewährleistet wird.

2.  Verfahren (100) nach vorhergehendem Anspruch, **dadurch gekennzeichnet, dass** es einen Schritt (1E4) des Erstellens eines Status des Schnitts in Abhängigkeit des Durchhangs $\alpha$ des Fadens und der ersten zeitlichen Ableitung $\dot\alpha$ dieses von jedem Sensor gemessenen Durchhangs $\alpha$ umfasst.

3.  Verfahren (100) nach vorhergehendem Anspruch, **dadurch gekennzeichnet, dass** es einen Schritt (1E5) zur Korrektur der Parameter des Schnitts und/oder einen Schritt zur Ausgabe einer Warnung in Abhängigkeit des Schnittstatus oder des Durchhangwerts FL und seiner ersten zeitlichen Ableitung umfasst.

4.  Verfahren (100) nach einem der beiden vorhergehenden Ansprüche, **dadurch gekennzeichnet, dass** der Status des Schnitts "nominal" ist, wenn bei jedem Sensor CA $\alpha$ kleiner ist als ein erster Schwellenwert $S_{1*}^{\alpha}$ und wenn bei jedem Sensor CA $\dot\alpha$ kleiner ist als ein zweiter Schwellenwert $SS_{2*}^{\dot\alpha}$, wenn der Schnitt um mehr als 15% fortgeschritten ist.

5.  Verfahren (100) nach einem der drei vorhergehenden Ansprüche, **dadurch gekennzeichnet, dass** der Status des Schnitts "riskant" ist, wenn bei einem und nur einem Sensor CA $\alpha$ größer ist als ein erster Schwellenwert $S_{1*}^{\alpha}$, und wenn bei einer vorbestimmten Anzahl von CA-Sensoren $\dot\alpha$ größer als ein zweiter Schwellenwert $S_{2*}^{\dot\alpha}$, und kleiner als ein dritter Schwellenwert $S_{3*}^{\dot\alpha}$ ist, wenn der Schnitt um mehr als 15% fortgeschritten ist.

6.  Verfahren (100) nach einem der vier vorhergehenden Ansprüche, **dadurch gekennzeichnet, dass** der Status des Schnitts "gefährlich" ist, wenn bei mindestens zwei CA-Sensoren $\alpha$ größer ist als ein erster Schwellenwert $S_{1*}^{\alpha}$, oder wenn bei mindestens zwei CA-Sensoren $\dot\alpha$ größer ist als ein dritter Schwellenwert $S_{3*}^{\dot\alpha}$, während der Schnitt um mehr als 10% fortgeschritten ist.

7.  Verfahren (100) nach einem der vorhergehenden Ansprüche, **dadurch gekennzeichnet, dass** es einen Schritt zur Bestimmung des Verhältnisses zwischen dem Durchhang (FL) und dem Schnittfortschritt oder der Schnittzeit umfasst, wobei die Verfolgung des Schnitts auch vom Wert des Verhältnisses abhängt.

8.  Schnittverfolgungsverfahren (200) in Abhängigkeit des Durchhangs (FL) von mindestens zwei Schneidfäden (FI) mit Hilfe von mindestens zwei Sensoren (CA), wobei der Faden (FI) beim Schneiden einen Durchhang (FL) aufweist und dieser Durchhang (FL) durch einen Winkel $\alpha$ gekennzeichnet werden kann, wobei das Verfolgungsverfahren (100) **dadurch gekennzeichnet ist, dass** es für jeden Sensor (CA) umfasst:

- einen Messschritt (2E1) des Durchhangs $\alpha$ des Fadens;
- einen Bestimmungsschritt (2E3) der ersten zeitlichen Ableitung $\dot\alpha$ und der zweiten $\ddot\alpha$ dieses Durchhangs $\alpha$;

wobei das Verfahren ferner einen Schritt (2E4) des Erstellens eines Status des Schnitts in Abhängigkeit des Durchhangs $\alpha$ des Fadens (FI), der ersten zeitlichen Ableitung $\dot\alpha$ und der zweiten zeitlichen Ableitung $\ddot\alpha$ dieses von jedem

Sensor (CA) gemessenen Durchhangs $\alpha$ umfasst, wobei der Status des Schnitts "gefährlich" ist, wenn bei mindestens zwei Sensoren (CA) $\alpha$ größer ist als der erste Schwellenwert $S_1^{\alpha}$ oder wenn bei mindestens zwei Sensoren (CA) $\ddot{\alpha}$ größer ist als der zweite Schwellenwert $S_2^{\ddot{\alpha}}$ und wenn bei diesen beiden Sensoren (CA) $\dot{\alpha}$ größer ist als ein fünfter Schwellenwert $S_5^{\dot{\alpha}}$, wobei diese Schritte (2E1, 2E3, 2E4) in regelmäßigen Abständen wiederholt werden, so dass eine Verfolgung des Schnitts gewährleistet wird.

9. Verfahren (200) nach vorhergehendem Anspruch, **dadurch gekennzeichnet, dass** es für jeden Sensor (CA) nach dem Messschritt (2E1) des Durchhangs $\alpha$ und vor dem Bestimmungsschritt (2E3) der ersten zeitlichen Ableitung $\dot{\alpha}$ und der zweiten zeitlichen Ableitung $\ddot{\alpha}$ dieses Durchhangs $\alpha$ einen Verarbeitungsschritt (2E2) der Messung des Durchhangs (FL) umfasst.

10. Verfahren (200) nach vorhergehendem Anspruch, **dadurch gekennzeichnet, dass** der Schnitt, dessen Verfolgung gewährleistet wird, mit einer Hin- und Rückbewegung des Schneidfadens (FI) durchgeführt wird, wobei die Hin- und Rückbewegung des Fadens (FI) mit einer bestimmten Frequenz durchgeführt wird, und dass der Verarbeitungsschritt (2E2) der Messung des Durchhangs (FL) einen Unterschritt zur Glättung der von den Sensoren stammenden Messung umfasst, wobei diese Glättung in Abhängigkeit der Frequenz der Hin- und Rückbewegung des Fadens (FL) durchgeführt wird.

11. Verfahren (200) nach einem der Ansprüche 8 bis 10, **dadurch gekennzeichnet, dass** es einen Schritt (2E5) zum Korrigieren der Parameter des Schnitts und/oder einen Schritt zum Ausgeben einer Warnung in Abhängigkeit vom Status des Schnitts umfasst.

12. Verfahren nach einem der Ansprüche 8 bis 11, **dadurch gekennzeichnet, dass** der Status des Schnitts "riskant" ist, wenn bei einem und nur einem Sensor (CA) $\alpha$ größer ist als der erste Schwellenwert $S_1^{\alpha}$, und wenn bei einer vorbestimmten Anzahl von Sensoren (CA) $\ddot{\alpha}$ kleiner ist als ein dritter Schwellenwert S3 und, wenn der Schnitt über einen vorbestimmten Schwellenwert hinaus vorangeschritten ist, wenn bei mindestens einem Sensor (CA) $\dot{\alpha}$ größer ist als ein vierter Schwellenwert $S_4^{\dot{\alpha}}$.

13. Verfahren nach einem der Ansprüche 8 bis 12, **dadurch gekennzeichnet, dass** es einen Schritt zur Bestimmung des Verhältnisses zwischen dem Durchhang (FL) und dem Schnittfortschritt oder der Schnittzeit umfasst, wobei die Schnittverfolgung auch vom Wert dieses Verhältnisses abhängt.

14. Schnittverfolgungsvorrichtung mit Mitteln zur Durchführung eines Verfahrens nach einem der vorhergehenden Ansprüche.

15. Computerprogramm mit Anweisungen, die die Vorrichtung nach dem vorhergehenden Anspruch dazu veranlassen, die Schritte des Verfahrens nach einem der Ansprüche 1 bis 13 auszuführen.

16. Computerlesbarer Träger, auf dem das Computerprogramm nach dem vorhergehenden Anspruch gespeichert ist.

**Claims**

1. Method (100) for monitoring cutting, the cutting being performed with a back-and-forth movement of the cutting wire (FI), the back and forth movement of the wire (FI) being performed at a determined frequency, the monitoring being performed as a function of the bow (FL) of at least one cutting wire (FI) using at least one sensor (CA), the wire (FI) having a bow (FL) during cutting, said bow (FL) being **characterised by** an angle $\alpha$, the method (100) comprising, for each sensor (CA):

   - a step (1E1) of measuring the bow $\alpha$ of the wire;
   - a step (1E2) of processing the measurement of the bow (FL) comprising smoothing the measurement from the sensor (CA), said smoothing being performed as a function of the frequency of the back-and-forth movement of the wire (FI);

- a step (1E3) of determining the first time derivative $\dot{\alpha}$: of the bow $\alpha$ from the measurement smoothed;

said steps (1E1, 1E2, 1E3) being reiterated at regular intervals so as to ensure cutting monitoring.

2. Method (100) according to the preceding claim, **characterised in that** it comprises a step (1E4) of establishing a cutting status as a function of the bow $\alpha$ of the wire and the first time derivative $\dot{\alpha}$: of said bow $\alpha$ measured by each sensor.

3. Method (100) according to the preceding claim, **characterised in that** it comprises a step (1E5) of correcting cutting parameters and/or a step of emitting an alert as a function of the cutting status or the value of the bow FL and its first time derivative.

4. Method (100) according to one of the two preceding claims, **characterised in that** the cutting status is "nominal" if, for each sensor CA, $\alpha$ is lower than a first threshold $S_{1*}^{\alpha}$ and if, for each sensor CA, $\dot{\alpha}$ is lower than a second threshold $S_{2*}^{\dot{\alpha}}$ when cutting is advanced by more than 15%.

5. Method (100) according to one of the three preceding claims, **characterised in that** the cutting status is "at risk" if, for one and a single sensor CA, $\alpha$ is greater than a first threshold $S_{1*}^{\alpha}$, and if, for a predetermined number of sensors CA, $\dot{\alpha}$ is greater than a second threshold $S_{2*}^{\dot{\alpha}}$ and lower than a third threshold $S_{3*}^{\dot{\alpha}}$ when cutting is advanced by more than 15%.

6. Method (100) according to one of the four preceding claims, **characterised in that** the cutting status is "dangerous" if, for at least two sensors CA, $\alpha$ is greater than a threshold $S_{1*}^{\alpha}$ or when, for at least two sensors CA, $\dot{\alpha}$ is greater than a third threshold $S_{3*}^{\dot{\alpha}}$ while cutting is advanced by more than 10%.

7. Method (100) according to one of the preceding claims, **characterised in that** it comprises a step of determining the ratio of the bow (FL) and the cutting advance or cutting time, cutting monitoring being also a function of the value of said ratio.

8. Method (200) for monitoring cutting as a function of the bow (FL) of at least two cutting wires (FI) using at least two sensors (CA), the wire (FI) having a bow (FL) during cutting, wherein said bow (FL) can be **characterised by** a angle $\alpha$, the monitoring method (200) being **characterised in that** it comprises, for each sensor (CA):

- a step (2E1) of measuring the bow $\alpha$ of the wire;
- a step (2E3) of determining the first $\dot{\alpha}$ and second $\ddot{\alpha}$ time derivatives of said bow $\alpha$;

the method further comprising a step (2E4) of establishing a cutting status as a function of the bow $\alpha$ of the wire (FI), of the first time derivative $\dot{\alpha}$ and of the second time derivative $\ddot{\alpha}$ of said bow $\alpha$ measured by each sensor (CA), the cutting status being "dangerous" when, for at least two sensors (CA), $\alpha$ is greater than the first threshold $S_{1}^{\alpha}$ or when, for at least two sensors (CA), $\ddot{\alpha}$ is greater than the second threshold $S_{2}^{\ddot{\alpha}}$ and if, for these two sensors (CA), $\dot{\alpha}$ is greater than a fifth threshold $S_{5}^{\dot{\alpha}}$, said steps (2E1, 2E3, 2E4) being reiterated at regular intervals so as to ensure cutting monitoring.

9. Method (200) according to the preceding claim, **characterised in that** it comprises, for each sensor (CA), after the step (2E1) of measuring the bow $\alpha$ and before the step (2E3) of determining the first $\dot{\alpha}$ and second $\ddot{\alpha}$ time derivatives of said bow $\alpha$, a step (2E2) of processing the measurement of the bow (FL).

10. Method (200) according to the preceding claim, **characterised in that** cutting which is monitored is performed with a back-and-forth movement of the cutting wire (FI), the back-and-forth movement of the wire (FI) being performed at a determined frequency and **in that** the step (2E2) of processing the measurement of the bow (FL) comprises a sub-step of smoothing the measurement from the sensors, said smoothing being performed as a function of the frequency of the back-and-forth movement of the wire (FL).

11. Method (200) according to one of claims 8 to 10, **characterised in that** it comprises a step (2E5) of correcting cutting parameters and/or a step of emitting an alert as a function of the cutting status.

12. Method according to one of claims 8 to 11, **characterised in that** the cutting status is "at risk" if, for one and a single sensor (CA), $\alpha$ is greater than the first threshold $S_1^{\alpha}$ and if, for a determined number of sensors (CA), $\ddot{\alpha}$ is lower than a third threshold $S_3^{\ddot{\alpha}}$, and when cutting is advanced beyond a predetermined advance threshold, if, for at least one sensor (CA), $\dot{\alpha}$ is greater than a fourth threshold $S_4^{\dot{\alpha}}$.

13. Method according to one of claims 8 to 12, **characterised in that** it comprises a step of determining the ratio of the bow (FL) to the cutting advance or cutting time, cutting monitoring being also a function of the value of said ratio.

14. Cutting monitoring device comprising means for implementing a method according to one of the preceding claims.

15. Computer program comprising instructions which cause the device according to the preceding claim to execute the steps of the method according to one of claims 1 to 13.

16. Computer readable medium, on which the computer program according to the preceding claim is recorded.

[Figure 1]

[Figure 2]

[Figure 3]

[Figure 4]

[Figure 5A]

[Figure. 5B]

[Fig. 6A]

[Figure 6B]

[Figure 7]

[Figure 8]

[Figure 9]

**EP 3 894 158 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2583778 A1
- US 6178961 B1
- EP 2708342 A1
- JP 2003127058 A
- CN 202428570 U
- CN 202507408 U